# EUROPÄISCHE PATENTSCHRIFT

(11) **EP 1 835 912 B1**
(45) Veröffentlichungstag und Bekanntmachung des Hinweises auf die Patenterteilung: **26.08.2009**
(21) Anmeldenummer: 05850375.6
(22) Anmeldetag: 29.12.2005
(51) Int. Cl.: A61K 31/4184, A61P 33/02

(54) **SUBSTITUIERTE BENZIMIDAZOLE ZUR BEHANDLUNG DER HISTOMIASIS**
SUBSTITUTED BENZIMIDAZOLES FOR TREATMENT OF HISTOMONIASIS
BENZIMIDAZOLES SUBSTITUES POUR TRAITER L'HISTOMONOSE

(30) Priorität: 05.01.2005 DE 102005000746
(43) Veröffentlichungstag der Anmeldung: 26.09.2007
(73) Patentinhaber: Bayer Animal Health GmbH, 51368 Leverkusen (DE)
(72) Erfinder: GREIF, Gisela, 53424 Remagen (DE); FROYMAN, Robrecht, 40789 Monheim (DE)
(86) Internationale Anmeldenummer: PCT/EP2005/014119
(87) Internationale Veröffentlichungsnummer: WO 2006/072448

(56) Entgegenhaltungen:
- WO-A-00/04022
- WO-A-00/68225
- ZENNER L ET AL: "IN VITRO EFFECT OF ESSENTIAL OILS FROM CINNAMOMUM AROMATICUM, CITRUS LIMON AND ALLIUM SATIVUM ON TWO INTESTINAL FLAGELLATES OF POULTRY, TETRATRICHOMONAS GALLINARUM AND HISTOMONAS MELEAGRIDIS" PARASITE, PRINCEPS EDITIONS, FR, Bd. 10, Nr. 2, 2003, Seiten 153-157, XP008034124 ISSN: 1252-607X
- MCDONALD: "OTHER PROTOZOAN DISEASES OF THE INTESTINAL TRACT" DISEASES OF POULTRY, 1991, Seiten 804-809,
- BONDURANT ET AL: "Histomonas meleagridis and Relatives" PARASITIC PROTOZOA, Bd. 9, 1994, Seiten 189-206,

## Beschreibung

Die vorliegende Erfindung betrifft die Verwendung von substituierten Benzimidazolen zur Behandlung der Histomoniasis bei Geflügel, insbesondere bei Puten.

Substituierte Benzimidazole und ihre Verwendung als Insektizide, Fungizide und Herbizide sind bereits bekannt geworden (EP-OS 87 375, 152 360, 181 826, 239 508, 260 744, 266 984, US-P 3 418 318, 3 472 865, 3 576 818, 3 728 994). Halogenierte Benzimidazole und ihre Wirkung als Anthelmintika, Coccidiostatika und Pestizide sind bekannt geworden (DE-OS 2 047 369, DE-OS 4 237 617). Die gemäß dieser Erfindung bevorzugt eingesetzten substituierten Benzimidazole sind beschrieben in WO 00/04022 und WO 00/68225.

Mischungen von nitrosubstituierten Benzimidazolen und Polyetherantibiotika sind als Coccidiosemittel bekannt geworden (US-P 5 331 003). Mischungen von substituierten Benzimidazolen mit Polyetherantibiotika oder synthetischen Coccidiosemitteln als Mittel zur Bekämpfung parasitärer Protozoen sind aus WO 96/38140 bekannt.

Histomoniasis ("Schwarzkopfkrankheit", "Blackhead Disease") ist eine Infektionskrankheit. Sie wird durch Histomonas spp., insbesondere Histomonas meleagridis verursacht, welcher zu den Darmparasiten zählt. Durch die Infektion kommt es zu einer schweren Blinddarm- und Leber-Entzündung, da der Erreger das Darmgewebe schädigt und über das Blut in die Leber gelangt, wo er eine Nekrosebildung bewirkt. Als Begleiterscheinung der Krankheit tritt oft Kreislaufversagen auf, kenntlich an den schwarz-blauen Köpfen erkrankter Tiere, denen die Krankheit ihren Namen verdankt.

In infizierten Betrieben breitet sich die Erkrankung sehr schnell auf den gesamten Bestand aus und führt durch sehr hohe Mortalitätsraten (in einigen Betreiben bis zu 100%) zu großen wirtschaftlichen Verlusten.

Histomonas meleagridis gehört aufgrund seiner strukturellen Geißeln zum Unterstamm der Flagellaten (Mastigophora). Die begeißelten Stadien vermehren sich im Blinddarm durch Zweiteilung. Amöboidartige Stadien dringen über die Blutbahn ausgehend vom infizierten Blinddarm in die Leber ein und zerstören diese über großflächige Nekrosen (BonDurant, R.H., Wakenell, P:S. (1994): Histomonas meleagridis and Relatives. In: Parasitic Protozoa, Volume 9, Chapter 3, pp 189-206. Academic Press)

Die Übertragung der Histomonaden auf direktem Weg, z.B. die orale Aufnahme von histomonadenhaltigem, frischen Kot, scheitert immer, da die Erreger außerhalb eines Wirtes nur für kurze Zeit lebensfähig sind und bei der Passage des Verdauungstraktes abgetötet werden. Tests amerikanischer Forscher (Hu and McDougald, 2003) ergaben, dass im Tierversuch eine Infektion bei Puten über die Kloake erfolgen kann. Da die Kloake nach dem Kotabsetzen einen leichten Sog erzeugt, ist eine Infektion innerhalb einer Herde auf diesem Weg unter praktischen Bedingungen, z.B. über verschmutzte Einstreu, wahrscheinlich. Wissenschaftlich nachgewiesen ist auch die Erregerübertragung über Zwischenwirte. Als Überträger (Transportvektor von Histomonas meleagridis) ist der Blinddarmwurm Heterakis gallinarum bekannt, vor allem die Heterakis-Eier oder Larvenstadien. Deswegen können Hühner und Puten sich bereits aktiv mit Histomonaden infizieren und erkranken, bevor erwachsene Heterakis-Würmer im Caecuminhalt erscheinen. In embryonierten Heterakis-Eiem können Histomonaden bis zu 4 Jahren infektiös bleiben. Weitere Zwischenwirte können Regenwürmer und Arthropoden sein, die mit Heterakis Eiern kontaminiert sind. Auch Hühner und andere Geflügelarten stellen eine potentielle Gefahr dar. Sie sind weniger empfindlich als Puten und tragen oftmals den Erreger in sich, ohne klinisch auffällig zu sein; so tragen sie zur Verbreitung des Erregers bei.

Puten können in jedem Lebensalter infiziert werden, am häufigsten tritt die Erkrankung jedoch zwischen der 3. und 12. Lebenswoche auf. Der Zeitraum zwischen Infektion und Krankheitsausbruch beträgt zumeist 7-12 Tage. Die Sterblichkeit kann bis zu 100% betragen und erreicht ihren höchsten Punkt am 17. Tag nach der Infektion. Ab dem 8. Tag sind Entzündungen im Blinddarm, ab dem 10. Tag in der Leber zu finden.

Infizierte Tiere sind matt und erschöpft, lassen Flügel und Köpfe hängen und verweigern die Futteraufnahme. Typisch ist das Absetzten von schwefelgelbem Kot, Durchfall und später auch Blutbeimischungen. Die mit der Krankheit einhergehenden Kreislaufstörungen verursachen eine ausgeprägte schwarz-blaue Färbung des Kopfes, daher der Name dieser Krankheit.

Der Verlauf der Erkrankung wird in erster Linie vom Alter und der Darmflora der Puten bestimmt. Zusätzliche Bakterieninfektionen mit E. coli, Clostridium perfringens oder Coccidien erschweren den Verlauf (McDougald, L.R., Hu, J. (2001): Blackhead Disease (Histomonas meleagridis) aggravated in broiler chickens by concurrent infection with cecal coccidiosis (Eimeria tenella). Avian Diseases 45:307-312)

Die Diagnose der Erkrankung kann über Nativpräparate aus Blinddarm und Leber mit Hilfe einer Kochsalzlösung erfolgen. Amöboid bewegliche Stadien sind im Phasenkontrastmikroskop sichtbar. Histologisch ist die PAS-Färbung im Gebrauch.

Bis 1950 waren Arsen-Verbindungen (z.B. Nitarson, Carbarson, Roxarson) die einzig wirksamen Verbindungen gegen Histomoniasis. Jedoch ist bekannt, dass Arsenverbindungen generell nicht stark genug sind, einmal etablierte Infektionen zu behandeln. Ein weiterer Nachteil ist ihr extrem geringer Sicherheitsindex: bereits eine 2fache Dosierung von Roxarson führt zu gestörten motorischen Funktionen im Truthahn.

Seit 1960 wurden Nitroimidazole und Nitrofurane intensiv in Futter- und/oder Wasser eingesetzt, jedoch Mitte der 90er Jahre zunehmend von der EU und der USA für die Anwendung im Nutztier und als Futterzusatzstoff verboten: Dimetridazol wurde 1997 in USA-vom Markt genommen und 2001 für die Nutzung als Futterzusatzstoff in der EU verboten. Seit dem 31.03.2003 darf nunmehr auch Nifursol, das einzige in der EU noch zugelassene Produkt, nicht mehr eingesetzt werden. Somit stehen derzeit und in Zukunft weder Arzneimittel für die Therapie noch Präparate zur prophylaktischen Bekämpfung der Schwarzkopfkrankheit zur Verfügung.

Helminthen wirksame Substanzen (Albendazol und Fenbendazol) sind in vitro gegen Histomonas unzureichend wirksam jedoch in vivo prophylaktisch wirksam, wenn 14 Tage lang ab Infektionszeitpunkt behandelt wird. Dabei richtet sich die Wirksamkeit nicht gegen H. meleagridis sondern gegen Heterakis gallinarum, den Transport Vektor von Histomonas meleagridis (Hu, J., McDougald, L.R., (2003): Direct lateral transmission of Histomonas meleagridis in turkeys. Avian Diseases 47:489-492).

Die derzeit einzig verfügbaren Strategien zur Vermeidung einer Erkrankung bestehen aus Maßnahmen zur Hygiene, Optimierung der Haltungsdichte und der Nährstoffversorgung sowie dem Vermeiden einer Erregerverschleppung. Diese Maßnahmen sind unzureichend und können eine Infektion und Erkrankung nicht verhindern,

Eine Impfung gegen Histomonas meleagridis ist biologisch nicht möglich, da auch eine natürliche Immunität nach einer Infektion nicht erworben werden kann. Einmal infizierte Tiere können erneut erkranken. Versuche über attenuierte Lebendimpfstoffe zu immunisieren waren erfolglos.

Es besteht daher der Bedarf nach Mitteln zu Behandlung der Histomoniasis.

Die Erfindung betrifft die Verwendung von Benzimidazolen der Formel (I) 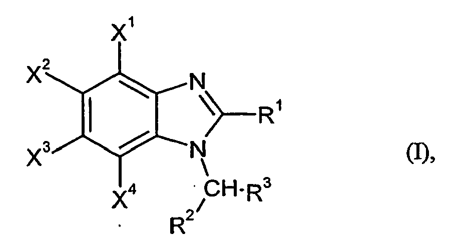 in welcher
- R¹: für Fluoralkyl steht,
- R²: für Wasserstoff oder Alkyl steht,
- R³: für einen Rest der Formel 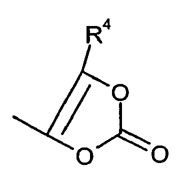 oder für einen Rest der Formel 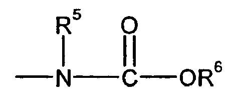 steht
- R⁴: für Alkyl steht,
- R⁵: für Alkyl oder substituiertes Phenyl steht,
- R⁶: für Alkyl steht,
- X¹,: X², X³ und X⁴ unabhängig voneinander für Wasserstoff, Halogen, Halogenalkyl, Halogenalkoxy, Halogenalkylthio oder Halogenalkylsulfonyl stehen,
oder auch
X² und X³ oder X³ und X⁴ gemeinsam für einen Dioxyhaloalkylen-Rest stehen,
zur Herstellung von Arzneimitteln zur Bekämpfung der Histomoniasis bei Geflügel.

Die erfmdungsgemäßen substituierten Benzimidazole sind durch die Formel (I) allgemein definiert.
- R¹: steht bevorzugt für C₁-C₄-Fluoralkyl,
- R²: steht bevorzugt für Wasserstoff oder C₁-C₄-Alkyl,
- R⁴: steht bevorzugt für C₁-C₄-Alkyl,

- R⁵: steht bevorzugt für C₁₋₆-Alkyl oder Phenyl, das gegebenenfalls ein- oder mehrfach substituiert ist mit C₁₋₄-Alkyl, C₁₋₄-Halogenalkyl, Halogen, Nitro, C₁₋₄-Alkoxy, C₁₋₄-Halogenalkoxy oder gegebenenfalls ein oder mehrfach mit Halogen substituiertes Methylen- oder Ethylendioxy.
- R⁶: steht bevorzugt für C₁₋₄-Alkyl
- X¹, X², X³ und X⁴: stehen bevorzugt unabhängig voneinander für Wasserstoff, F, Cl, Br, C₁-C₄-Halogenalkyl, C₁-C₄-Halogenalkoxy, C₁-C₄-Halogenalkylthio, C₁-C₄-Halogenalkylsulfonyl, oder
- X²: und X³ oder X³ und X⁴ stehen gemäß einer weiteren bevorzugten Ausführungsform gemeinsam für einen Dioxyhalo-C₁-C₄-alkylenrest.
- R¹: steht besonders bevorzugt für CF₃, CHF₂ oder CHF.
- R²: steht besonders bevorzugt für Wasserstoff, Methyl, Ethyl, n-Propyl oder Isopropyl.
- R⁴: steht besonders bevorzugt für Methyl, Ethyl, n-Propyl oder Isopropyl.
- R⁵: steht besonders bevorzugt für C₁₋₆-Alkyl.
- R⁶: steht besonders bevorzugt für Methyl oder Ethyl.
- X¹, X², X³ und X⁴: stehen besonders bevorzugt unabhängig voneinander für Wasserstoff, F, Cl, Br, CF₃, CHF₂, CH₂F, OCF₃, OCH₂F, OCHF₂, SCF₃, SCHF₂, SCH₂F, SO₂CF₃, SO₂CHF₂, SO₂CH₂F.
- X²und X³ oder X³ und X⁴: stehen gemäß einer weiteren Ausführungsform besonders bevorzugt auch gemeinsam für einen Rest -O-CF₂-O-, -O-CF₂-CF₂-O -O-CF₂-CF₂-CF₂-O-, -O-CF₂-CHF-O-, -O-CClF-CClF-O-, -O-CHF-O-, -O-CHF-CHF-O- oder -O-CClF-O-.

Gemäß einer ganz besonders bevorzugten Ausführungsform steht R³ für einen Rest der Formel

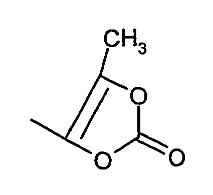

Gemäß einer weiteren ganz besonders bevorzugten Ausführungsform steht R³ für einen Rest der Formel 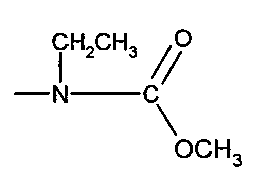
- R¹: steht ganz besonders bevorzugt für-CF₃.
- R²: steht ganz besonders bevorzugt für Wasserstoff.
- R⁴: steht ganz besonders bevorzugt für Methyl.
- X¹: steht ganz besonders bevorzugt für Cl oder Br.
- X²: steht ganz besonders bevorzugt für Wasserstoff.
und
- X³: und X⁴ stehen ganz besonders bevorzugt gemeinsam für -OCF₂-CF₂-O-.

Alkyl steht für einen geradkettigen oder verzweigten Kohlenwasserstoffrest mit 1 bis 8, bevorzugt 1 bis 6, besonders bevorzugt 1 bis 4 Kohlenstoffatomen, wie z. B. Methyl, Ethyl, Propyl, Isopropyl, n-Butyl, sek.-Butyl, tert-Butyl.

Alkylen steht für einen geradkettigen oder verzweigen Kohlenwasserstoffrest mit 1 bis 4, bevorzugt 1 bis 3 besonders bevorzugt 1 bis 2 Kohlenstoffatomen, der über zwei verschiedene Positionen verknüpft ist.

Halogenalkyl steht für einen Alkylrest, wie oben definiert, in dem ein oder mehrere, insbesondere 1 bis 3 Wasserstoffatome durch ein Halogenatom, insbesondere Fluor, Chlor oder Brom ersetzt wurden.

Fluoralkylrest steht entsprechend für einen Alkylrest in dem 1 bis alle Wasserstoffatome durch Fluoratome ersetzt wurden; bevorzugt sind Perfluoralkylreste, z. B. Trifluormethyl oder Pentafluorethyl.

Halogenalkoxy steht für einen geradkettigen oder verzweigten Alkoxyrest mit 1 bis 8, bevorzugt 1 bis 6, besonders bevorzugt 1 bis 4 Kohlenstoffatomen, in dem ein oder mehrere, insbesondere 1 bis 3 Wasserstoffatome durch ein Halogenatom, insbesondere Fluor, Chlor oder Brom ersetzt wurden; z. B. -OCF₃.

Halogenalkylthio steht für einen geradkettigen oder verzweigten Alkylthio-Rest mit 1 bis 8, bevorzugt 1 bis 6, besonders bevorzugt 1 bis 4 Kohlenstoffatomen, in dem ein oder mehrere, insbesondere 1 bis 3 Wasserstoffatome durch ein Halogenatom, insbesondere Fluor, Chlor oder Brom ersetzt wurden; z.B. CF₃S-.

Halogenalkylsulfonyl steht für einen geradkettigen oder verzweigten Alkylsulfonylrest mit 1 bis 8, bevorzugt 1 bis 6, besonders bevorzugt 1 bis 4 Kohlenstoffatomen, in dessen Alkylteil ein oder mehrere, insbesondere 1 bis 3 Wasserstoffatome durch ein Halogenatom, insbesondere Fluor, Chlor oder Brom ersetzt wurden.

Beispiele für erfindungsgemäß besonders bevorzugte substituierte Benzimidazole sind die Verbindung der Formel (I-1) (siehe WO00/04022) und insbesondere die Verbindung der Formel (I-2) (siehe WO00/68225):

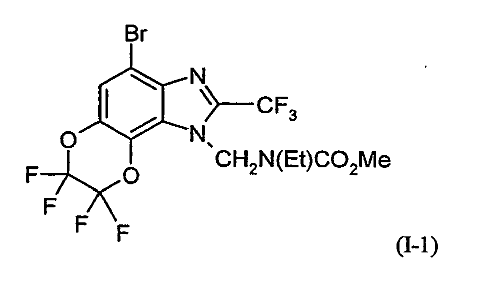

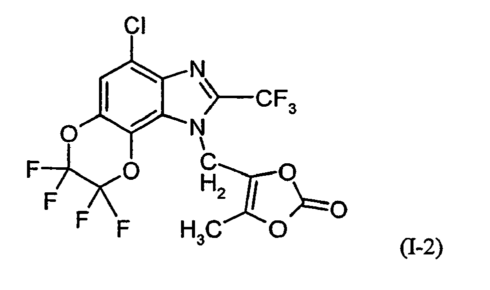

Die vorstehend genannten Wirkstoffe können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Sowohl die reinen Isomeren als auch die Isomerengemische können erfindungsgemäß eingesetzt werden.

Soweit die Wirkstoffe Salze bilden können kommt auch der Einsatz in Form pharmazeutisch verträglicher Salze in Frage.

Weiterhin kommt gegebenenfalls auch der Einsatz von Hydraten oder anderen Solvaten der Wirkstoffe oder ihrer Salze in Frage.

Die Anwendung kann sowohl prophylaktisch als auch therapeutisch erfolgen.

Histomoniasis wird hervorgerufen durch Histomonas spp.. Bevorzugt ist die erfmdungsgemäße Bekämpfung der Histomoniasis hervorgerufen durch Histomonas meleagridis.

Erfindungsgemäß behandelt wird üblicherweise Geflügel, wie z. B. Hühner, Wachteln, Enten, Gänse, Fasane und insbesondere Puten (hier synonym mit Truthähnen).

Die Anwendung der Wirkstoffe erfolgt direkt oder in Form von geeigneten Zubereitungen enteral, parenteral, dermal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden, wie oben bei den Injektionslösungen beschrieben, hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden, wie oben bei den Injektionslösungen beschrieben, hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, dass eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole,Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder von Typ Öl in Wasser.

Sie werden hergestellt, indem man die Wirkstoffe entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge C₈₋₁₂ oder anderen speziell ausgewählten natürlichenFettsäuren, Partialglyceridgemische gesättigter oder ungesättigter, eventuell auch hydroxylgruppenhaltiger Fettsäuren, Mono- und Diglyceride der C₈/C₁₀ -Fettsäuren.

Fettsäureester wie Ethylstearat, Di-n-butyryl-adipat, Laurinsäurehexylester, Dipropylenglykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt:
nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt:
Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen werden die Wirkstoffe mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in Kombination mit Synergisten oder mit weiteren Wirkstoffen vorliegen.

Als weitere Wirkstoffe kommen z.B.in Frage:
Coccidiostatika, wie Robenidin oder Amprolium, z.T. in Kombination mit Folsäureantagonisten; Polyetherantibiotika wie Monensin, Salinomycin, Lasalocid, Narasin, Semduramicin oder insbesondere Maduramicin;
Triazinone, wie Toltrazuril, Ponazuril oder Diclazuril;
Sulfonamide;
Anthelinintika, z. B. Febantel, Benzimidazol-Anthelmintika oder Depsipeptid-Anthelmintika wie PF 1022 A oder Emodepside.

Anwendungsfertige Zubereitungen enthalten die Wirkstoffe jeweils in Konzentrationen von 0,005 ppm bis 50 ppm, bevorzugt von 0,1 bis 10 ppm.

Im Allgemeinen hat es sich als vorteilhaft erwiesen, Mengen von etwa 0,05 bis etwa 50 mg, bevorzugt 0,1 bis 20 mg, Wirkstoff je kg Körpergewicht pro Tag zur Erzielung wirksamer Ergebnisse zu verabreichen.

In der Mischung mit anderen Coccidiosemitteln oder Polyetherantibiotika liegen die erfindungsgemäßen Wirkstoffe im Verhältnis 1 zu 0,01 - 50 bis 1 zu 1 - 50 vor.

Die Wirkstoffe können auch zusammen mit dem Futter oder Trinkwasser der Tiere verabreicht werden.

Futter- und Nahrungsmittel enthalten 0,005 bis 250 ppm, vorzugsweise 0,05 bis 100 ppm des Wirkstoffs in Kombination mit einem geeigneten essbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem essbaren organischen oder anorganischen Träger enthält mit üblichen Futtermitteln. Essbare Träger sind z.B. Maismehl oder Mais- und Sojabohnenmehl oder Mineralsalze, die vorzugsweise eine geringe Menge eines essbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Histomoniasis beschrieben:
Für die Heilung und Prophylaxe der Histomoniasis bei Geflügel, insbesondere bei Hühnern, Enten, Gänsen oder Truthähnen, werden 0,005 bis 100 ppm, vorzugsweise 0,05 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Trotzdem kann es zeitweilig notwendig sein, von den genannten Mengen abzuweichen, insbesondere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muss. Bei der Verabreichung größerer Mengen kann es zweckmäßig sein, diese im Verlauf des Tages in mehrere Einzeldarreichungen zu unterteilen.

Die Wirksamkeit der erfindungsgemäßen Verbindungen lässt sich z.B. in Käfigversuchen mit folgender Versuchsanordnung belegen, bei der die Tiere mit dem jeweiligen Wirkstoff behandelt werden.

Ein wirkstoffhaltiges Futter wird so zubereitet, dass die erforderliche Menge Wirkstoff mit einem nährstoffmäßig ausgeglichenen Tierfutter, z.B. mit dem unter angegebenen Kükenfutter, gründlich vermischt wird.

Wenn ein Konzentrat oder eine Vormischung zubereitet werden soll, die schließlich im Futter auf die im Versuch genannten Werte verdünnt werden soll, werden im Allgemeinen etwa 1 bis 30 %, vorzugsweise etwa 10 bis 20 Gew.-% Wirkstoff mit einem essbaren organischen oder anorganischen Träger, z.B. Mais- und Sojamehl oder Mineralsalzen, die eine kleine Menge eines essbaren Entstäubungsöls, z.B. Maisöl oder Sojabohnenöl enthalten, vermischt. Die so erhaltene Vormischung kann dann dem vollständigen Geflügelfutter vor der Verabreichung zugegeben werden.

Als Beispiel für die Verwendung der erfindungsgemäßen Stoffe im Geflügelfutter kommt die folgende Zusammensetzung in Frage.

| | |
|---|---|
| 52,00 % | Futtergetreideschrot, und zwar: 40 % Mais, 12 % Weizen |
| 17,00 % | Sojaschrot extr. |
| 5,00 % | Maisklebefutter |
| 5,00 % | Weizenfuttermehl |
| 3,00 % | Fischmehl |
| 3,00 % | Mineralstoffinischung |
| 3,00 % | Luzernegrasgrünmehl |
| 2,50 % | Vitaminvormischung |
| 2,00 % | Weizenkeime, zerkleinert |
| 2,00 % | Sojaöl |
| 2,00 % | Fleischknochenmehl |
| 1,50 % | Molkenpulver |
| 1,00 % | Melasse |
| 1,00 % | Bierhefe, gebunden an Biertreber |
| 100,00 % | |

Ein solches Futter enthält 18 % Rohprotein, 5 % Rohfaser, 1 % Ca, 0,7 % P sowie je kg 1200 i.E. Vitamin A, 1200 i.E. Vitamin D3, 10 mg Vitamin E, 20 mg Zinkbacitracin.

### Biologisches Beispiel:

Junge, anfällige Truthahnküken wurden in Käfigen gehalten und in der üblichen Weise intrakloakal im Alter von 2 Wochen mit etwas 5 log10 infektiösen Histomonas meleagridis Mikroorganismen infiziert. Bei den Testgruppen, die mit den Testverbindungen behandelt worden waren, wurden zwei Kontrollgruppen zu Vergleichszwecken gehalten (infiziert/unbehandelt und nicht infiziert/unbehandelt). Die Testgruppen bestanden aus 6 bis 10 Puten. Den behandelten Testgruppen wurden die Testverbindungen, entweder die Verbindung der Formel (I-1) oder (I-2), 1 Tag vor der Infektion und 13 Tage danach mit dem Futter verabreicht. Beim Versuchsende wurden die Tiere getötet und auf Läsionen untersucht, die für Histomoniasis typisch sind ("Schwarzkopfkrankheit"). Die Ergebnisse der zwei Versuche sind in den Tabellen 1 und 2 zusammengefasst.

**Tabelle 1**

| **Gruppe** | **Test 1** | **Gewichtszunahme*** |
|---|---|---|
| 1 | 10 ppm Verbindung (I-2) im Futter | 35 |
| 2 | 5 ppm Verbindung (I-1) im Futter | 61 |
| 3 | Infizierte/unbehandelte Kontrolle | 7 |
| 4 | nichtinfizierte/unbehandelte Kontrolle | 100 |

| | | |
|---|---|---|
| * Gewichtszunahme der Gruppe 4 wurde auf 100 % gesetzt | | |

**Tabelle 2**

| **Gruppe** | **Test 2** | **Leberläsionen*** | **Caecumläsionen*** |
|---|---|---|---|
| 1 | 10 ppm Verbindung (I-2) im Futter | 0 | 2,1 |
| 1 | 30 ppm Verbindung (I-2) im Futter | 0 | 0,8 |
| 2 | 5 ppm Verbindung (I-1) im Futter | 0,8 | 3,5 |
| 2 | 15 ppm Verbindung (I-1) im Futter | 0 | 3 |
| 3 | Infizierte/unbehandelte Kontrolle | 1,8 | 3,8 |
| 4 | nichtinfizierte/unbehandelte Kontrolle | 0 | 0 |

| | | | |
|---|---|---|---|
| * Bewertungsskala von 0 (keine Läsionen), 1, 2, 3 bis 4 (schwerste Läsionen) | | | |

Es konnte gezeigt werden, dass die Testverbindungen gegen die Auswirkungen der Histomonas meleagridis Infektion klinisch wirksam sind. Im Test 1 verursachte Histomonas meleagridis schwere Wachstumsstörungen und die Testverbindungen schwächten diese Auswirkungen ab.

Im Test 2 verursachte Histomonas meleagridis deutliche Leber- und Caecum-Läsionen mit anschließender Schwarzkopfkrankheit. Beide Testverbindungen reduzierten diese Leberläsionen, welche die Krankheit verursachen, deutlich. Die Verbindung der Formel (I-2) verringerte auch die Caecumläsionen.

## Patentansprüche

1. Verwendung von Benzimidazolen der Formel (I) 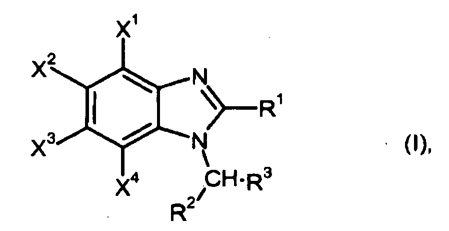 in welcher
R¹ für Fluoralkyl steht,
R² für Wasserstoff oder Alkyl steht,
R³ für einen Rest der Formel 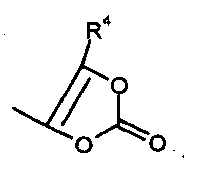 oder für einen Rest der Formel 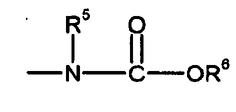 steht
R⁴ für Alkyl steht,
R⁵ für Alkyl oder substituiertes Phenyl steht,
R⁶ für Alkyl steht,
X¹, X², X³ und X⁴ unabhängig voneinander für Wasserstoff, Halogen, Halogenalkyl, Halogenalkoxy, Halogenalkylthio oder Halogenalkylsulfonyl stehen,
oder auch
X² und X³ oder X³ und X⁴ gemeinsam für einen Dioxyhaloalkylen-Rest stehen,
zur Herstellung von Arzneimitteln zur Bekämpfung der Histomoniasis bei Geflügel.

2. Verwendung gemäß Anspruch 1 von Verbindungen der Formel (I), worin
R¹ für C₁-C₄-Fluoralkyl steht,
R² für Wasserstoff oder C₁-C₄-Alkyl steht,
R⁴ für C₁-C₄-Alkyl steht,
R⁵ für C₁₋₆-Alkyl oder Phenyl, das gegebenenfalls ein- oder mehrfach substituiert ist mit C₁₋₄-Alkyl, C₁₋₄-Halogenalkyl, Halogen, Nitro, C₁₋₄-Alkoxy, C₁₋₄-Halogenalkoxy oder gegebenenfalls ein oder mehrfach mit Halogen substituiertes Methylen- oder Ethylendioxy steht,
R⁶ für C₁₋₄-Alkyl steht,
X¹, X², X³ und X⁴ unabhängig voneinander für Wasserstoff, F, Cl, Br, C₁-C₄-Halogenalkyl, C₁-C₄-Halogenalkoxy, C₁-C₄-Halogenalkylthio, C₁-C₄-Halogenalkylsulfonyl stehen, oder
X² und X³ oder X³ und X⁴ gemeinsam für einen Dioxyhalo-C₁-C₄-alkylenrest.stehen.

3. Verwendung gemäß Anspruch 1 der Verbindung der Formel (I-1)

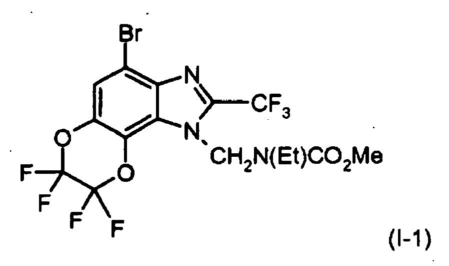

4. Verwendung gemäß Anspruch 1 der Verbindung der Formel (I-2)

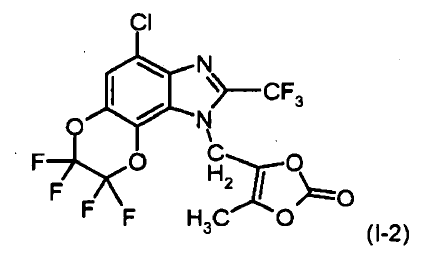

5. Verwendung gemäß einem der vorstehenden Ansprüche zur Bekämpfung der Histomoniasis hervorgerufen durch Histomonas meleagridis.

6. Verwendung gemäß einem der vorstehenden Ansprüche zur Bekämpfung der Histomoniasis bei Puten.

7. Benzimidazole der Formel (I), definiert wie in einem der Ansprüche 1 bis 4, zur Verwendung zur Bekämpfung der Histomoniasis bei Geflügel.

## Claims

1. Use of benzimidazoles of the formula (I) 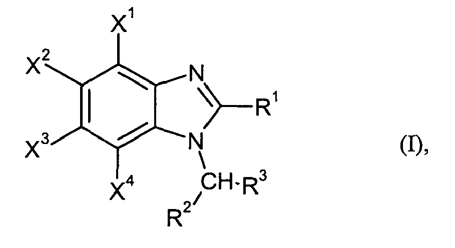 in which
R¹ is fluoroalkyl,
R² is hydrogen or alkyl,
R³ is a radical of the formula 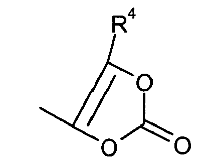 or is a radical of the formula 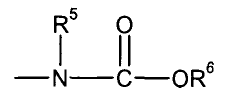
R⁴ is alkyl,
R⁵ is alkyl or substituted phenyl,
R⁶ is alkyl,
X¹ X², X³ and X⁴ are independently of one another hydrogen, halogen, haloalkyl, haloalkoxy, haloalkylthio or haloalkylsulphonyl,
or else
X² and X³ or X³ and X⁴ together are a dioxyhaloalkylene radical,
for manufacturing medicaments for controlling histomoniasis in poultry.

2. Use according to Claim 1 of compounds of the formula (I) in which
R¹ is C₁-C₄-fluoroalkyl,
R² is hydrogen or C₁-C₄-alkyl,
R⁴ is C₁-C₄-alkyl,
R⁵ is C₁₋₆-alkyl or phenyl which is optionally substituted one or more times by C₁₋₄-alkyl, C₁₋₄-haloalkyl, halogen, nitro, C₁₋₄-alkoxy, C₁₋₄-haloalkoxy or optionally mono- or poly-halogen-substituted methylene- or ethylenedioxy,
R⁶ is C₁₋₄-alkyl,
X¹, X², X³ and X⁴ are independently of one another hydrogen, F, Cl, Br, C₁-C₄-haloalkyl, C₁-C₄-haloalkoxy, C₁-C₄-haloalkylthio, C₁-C₄-haloalkylsulphonyl, or
X² and X³ or X³ and X⁴ together are a dioxyhalo-C₁-C₄-alkylene radical.

3. Use according to Claim 1 of the compound of the formula (I-1)

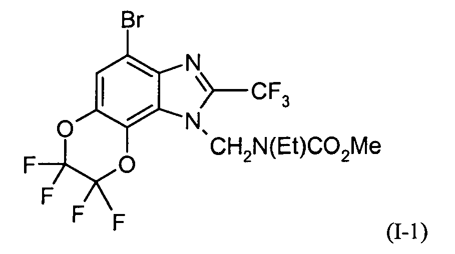

4. Use according to Claim 1 of the compound of the formula (I-2)

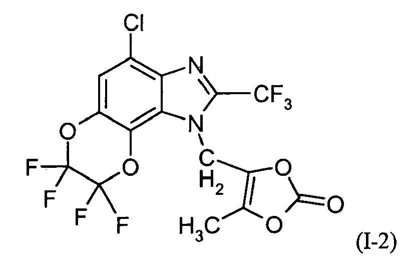

5. Use according to any of the preceding claims for controlling histomoniasis caused by Histomonas meleagridis.

6. Use according to any of the preceding claims for controlling histomoniasis in turkeys.

7. Benzimidazoles of the formula (I) as defined in any of Claims 1 to 4 for use for controlling histomoniasis in poultry.

## Revendications

1. Utilisation de benzimidazoles de formule (I) 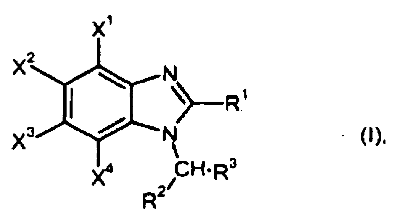 dans laquelle
R¹ est un reste fluoralkyle,
R² est l'hydrogène ou un reste alkyle
R³ représente un reste de formule 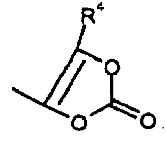 ou un reste de formule 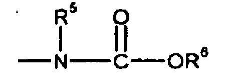
R⁴ est un reste alkyle,
R⁵ est un reste alkyle ou un reste phényle substitué,
R⁶ est un reste alkyle
X¹, X², X³ et X⁴ représentent, indépendamment les uns des autres, l'hydrogène, un halogène, un reste halogénalkyle, halogénalkoxy, halogénalkylthio ou halogénalkylsulfonyle,
ou bien
X² et X³ ou X³ et X⁴ forment ensemble un reste dioxyhalogénalkylène,
pour la préparation de médicaments destinés à combattre l'histomonose de la volaille.

2. Utilisation suivant la revendication 1 de composés de formule (I) dans laquelle
R¹ est un reste fluoralkyle en C₁ à C₄,
R² est l'hydrogène ou un reste alkyle en C₁ à C₄,
R⁴ est un reste alkyle en C₁ à C₄,
R⁵ est un reste alkyle en C₁ à C₆ ou un reste phényle, qui est éventuellement substitué une ou plusieurs fois avec un radical alkyle en C₁ à C₄, halogénalkyle en C₁ à C₄, halogéno, nitro, alkoxy en C₁ à C₉, halogénalkoxy en C₁ à C₄ ou un reste méthylènedioxy ou éthylènedioxy éventuellement substitué une ou plusieurs fois avec un radical halogéno,
R⁶ est un reste alkyle C₁ à C₄,
X¹, X², X³ et X⁴ représentent, indépendamment les uns des autres, l'hydrogène, F, Cl, Br, un reste halogénalkyle en C₁ à C₄ , halogénalkoxy en C₁ à C₄, halogénalkylthio en C₁ à C₄, halogénalkylsulfonyle en C₁ à C₄, ou bien
X² et X³ ou X³ et X⁴ forment ensemble un reste dioxyhalogénalkylène en C₁ à C₄.

3. Utilisation suivant la revendication 1 du composé de formule (I-1)

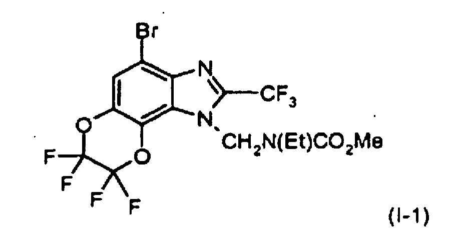

4. Utilisation suivant la revendication 1 du composé de formule (1-2)

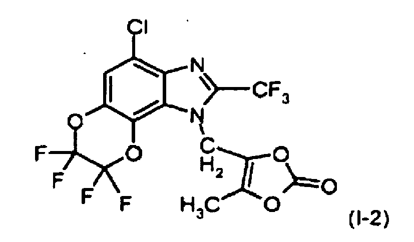

5. Utilisation suivant l'une des revendications précédentes pour combattre l'histomonose causée par Histomonas meleagridis.

6. Utilisation suivant l'une des revendications précédentes pour combattre l'histomonose de la dinde.

7. Benzimidazoles de formule (I) telle que définie dans l'une des revendications 1 à 4, destinés à être utilisés pour combattre l'histomonose de la volaille.
